# EUROPÄISCHE PATENTANMELDUNG

(11) **EP 0 943 340 A1**
(43) Veröffentlichungstag der Anmeldung: **22.09.1999**
(21) Anmeldenummer: 99104859.6
(22) Anmeldetag: 11.03.1999
(51) Int. Cl.: A61K 47/34, A61K 7/00, A23L 1/035

(54) **Verwendung von polymerisierten Fettsäurederivaten und Fettalkoholderivaten als Solubilisatoren**

(30) Priorität: 20.03.1998 DE 19812152
(71) Anmelder: BASF AKTIENGESELLSCHAFT, 67056 Ludwigshafen (DE)
(72) Erfinder: Dralle-Voss, Gabriele, Dr., 64665 Alsbach-Hähnlein (DE); Ruchatz, Folker, Dr., 67433 Neustadt (DE); Zirnstein, Michael, Dr., 69198 Schriesheim (DE); Oppenländer, Knut, Dr., 67061 Ludwigshafen (DE); Kolter, Karl, Dr., 67117 Limburgerhof (DE)

(57) **Zusammenfassung**

Verwendung von polymerisierten Fettsäure- und Fettalkoholderivaten, hergestellt durch Kondensation von polymerisierten C₁₂-C₁₁₀-Fettsäuren oder C₁₂-C₁₁₀-Fettalkoholen mit hydrophilen Bausteinen aus der Gruppe, bestehend aus Polyalkoholen, Monosacchariden, Alkylenglykolen, Polyalkylenglykolen, aminierten Polyalkylenglykolen, als Solubilisatoren.

## Beschreibung

Die Erfindung betrifft die Verwendung von polymerisierten Fettsäurederivaten und Fettalkoholderivaten als Solubilisator.

Bei der Herstellung homogener pharmazeutischer oder kosmetischer Zubereitungen hat die Solubilisierung von hydrophoben Stoffen eine sehr große praktische Bedeutung erlangt.

Unter Solubilisierung ist eine Löslichkeitsverbesserung durch oberflächenaktive Verbindungen zu verstehen, die in der Lage sind, schlecht wasserlösliche oder wasserunlösliche Stoffe in klare, höchstens opaleszierende wäßrige Lösungen zu überführen, ohne daß hierbei die chemische Struktur dieser Stoffe eine Veränderung erfährt.

Die hergestellten Solubilisate sind dadurch gekennzeichnet, daß der schlecht wasserlösliche oder wasserunlösliche Stoff in den Molekülassoziaten der oberflachenaktiven Verbindungen, die sich in wäßriger Lösung bilden - den sogenannten Mizellen - gelöst vorliegt. Die resultierenden Lösungen sind stabile einphasige Systeme, die optisch klar bis opaleszent erscheinen und ohne größeren Energieeintrag hergestellt werden können.

Solubilisatoren können das Aussehen beispielsweise von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Als Solubilisatoren für pharmazeutische Arzneistoffe und kosmetische Wirkstoffe werden hauptsächlich folgende Produkte eingesetzt:
- ethoxiliertes (hydriertes) Ricinusöl, (z.B. Cremophor® Marken, Fa. BASF);
- ethoxilierte Sorbitanfettsäureester, (z.B. Tween® Marken, Fa. ICI);
- ethoxilierte Hydroxystearinsäure, (z.B. Solutol® Marken, Fa. BASF).

Die oben beschriebenen, bisher eingesetzten Solubilisatoren zeigen jedoch eine Reihe anwendungstechnischer Nachteile.

So ist z. B. deren parenterale Applikation mit einer Freisetzung von Histamin und einem daraus resultierenden Blutdruckabfall verbunden (Lorenz et Al., Agents and Actions, Vol. 12, 1/2, 1982).

Die bekannten Solubilisatoren besitzen für einige schwerlösliche Arzneistoffe z. B. Clotrimazol nur eine geringe lösungsvermittelnde Wirkung.

Oberflächenaktive Verbindungen besitzen häufig eine hohe hämolytische Aktivität, die einer Anwendung auf dem Gebiet der Pharmazie, insbesondere in Parenteralia entgegensteht.

EP-A-0 131 558 beschreibt Ester aus polymerisierten Fettsäuren und Polyethylenglykol bzw. einseitig verschlossenem Polyethylenglykol als Dispergiermittel für feste hydrophobe Partikel in Wasser.

EP-A-0 229 400 beschreibt Polyester aus polymerisierten Fettsäuren, Polyethylenglykolen und Fettsäuren als Verdickungsmittel für tensidhaltige, kosmetische und pharmazeutische Präparate.

Es bestand nun die Aufgabe, neue Solubilisatoren für pharmazeutische, kosmetische sowie lebenmitteltechnische Anwendungen bereitzustellen, die die oben genannten Nachteile nicht aufweisen.

Diese Aufgabe wurde erfindungsgemäß gelöst durch die Verwendung von polymerisierten Fettsäure- und Fettalkoholderivaten, hergestellt durch Kondensation von polymerisierten C₁₂-C₁₁₀-Fettsäuren oder C₁₂-C₁₁₀-Fettalkoholen mit hydrophilen Verbindungen aus der Gruppe, bestehend aus Polyalkoholen, Monosacchariden, Alkylenglykolen, Polyalkylenglykolen und aminierten Polyalkylenglykolen als Solubilisatoren.

Unter polymerisierten Fettsäuren sind gesättigte oder ungesättigte Fettsäuren mit 12 bis 110 C-Atomen, bevorzugt 24 bis 44 C-Atomen, besonders bevorzugt 32 bis 40 C-Atomen zu verstehen, die durch Polymerisation einer oder verschiedener ungesättigter Fettsäuren hergestellt werden.

Die polymerisierbaren Fettsäuren bzw. Fettalkohole sind einfach oder mehrfach ungesättigte Verbindungen mit einer Kohlenstoffkette von 6 bis 22 C-Atomen, bevorzugt 12 bis 22 C-Atomen, besonders bevorzugt 16 bis 20 C-Atomen sowie Gemische dieser Fettsäuren bzw. Fettalkohole, beispielsweise Ölsäure - Linolsäure Gemische.

Die ebenfalls beanspruchten polymerisierten Fettalkohole mit 12 bis 110 C-Atomen, bevorzugt 24 bis 44 C-Atomen, besonders bevorzugt 32 bis 40 C-Atomen können durch Hydrierung der entsprechenden polymerisierten Fettsäuren erhalten werden und liegen bevorzugt in der gesättigten Form vor.

Die Polymerisation der Fettsäuren bzw. Fettalkohole kann zu dimeren, trimeren bis hin zu pentameren Strukturen führen.

Bevorzugte Verwendung als Solubilisator finden dimere und trimere Fettsäure- oder Fettalkoholderivate, insbesondere dimere Fettsäurederivate.

Die dimerisierten Derivate enthalten im wesentlichen lineare und cyclische Verbindungen, die ungesättigt oder hydriert sein können, bevorzugt aber hydriert sind.

Beispiele für ungesättigte Dimerfettsäurestrukturen:

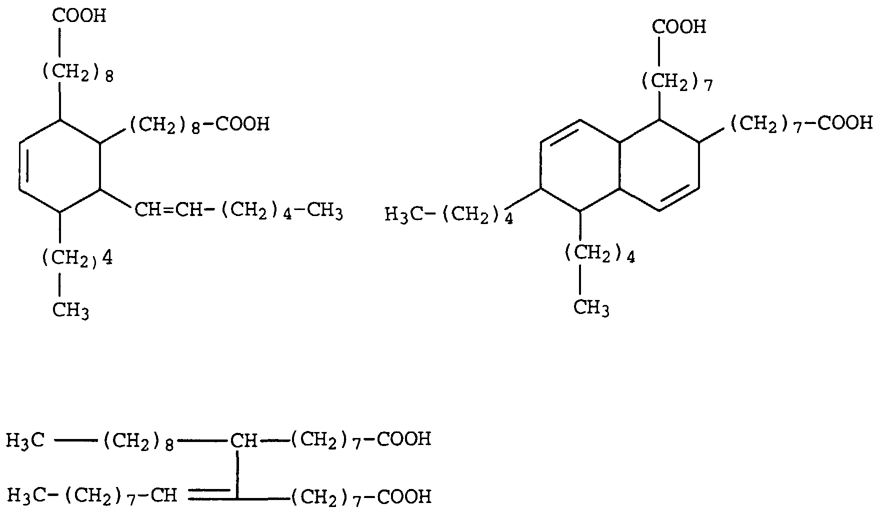

Als polymerisierte Fettsäuren kommen vorzugsweise die Produkte in Frage, die unter der Bezeichnung Pripol® (Fa. Unichema) oder Empol® (Fa. Henkel) im Handel erhältlich sind. Diese dimerisierten Öl-/Linolsäure Gemische enthalten vorwiegend lineare und cyclische Verbindungen. Daneben können diese Produkte auch noch Anteile von monomeren sowie von trimeren und höher kondensierten Fettsäuren enthalten.

Typische im Handel erhältliche dimere Fettsäuren haben etwa folgende Zusammensetzung:
- Monomere Säuren:: 0-15 Gew.-%,
- dimere Säuren:: 50-99 Gew.-%,
- tri- und höherpolymerisierte Säuren:: 1-35 Gew.-%,
wobei der Gehalt je nach Herkunft der Monomeren, des Polymerisationsverfahrens sowie des Aufarbeitungsprozesses innerhalb dieser Grenzen schwanken kann.

Als hydrophile, für die Kondensation mit den polymerisierten Fettsäuren oder Fettalkoholen geeignete Verbindungen seien genannt:

Polyalkohole, z.B. Pentaerythrit, Glycerin, Oligoglycerine mit 2-20 Einheiten, Zuckeralkohole wie D-Sorbit und D-Mannit; Alkylenglykole, insbesondere C₂-C₄-Alkylenglykole, beispielsweise Ethylenglykol, Methylethylenglykol, Propylenglykol sowie deren Dimere;

Polyalkylenglykole, z.B. Polyethylenglykole, Polypropylenglykole, gegebenenfalls aminiert, mit einem Molekulargewicht von 200 bis 12000 g/mol, bevorzugt 300 bis 5000 g/mol, besonders bevorzugt 400 bis 1500 g/mol;

Einseitig verschlossene Polyalkylenglykole, z.B. Methylpolyethylenglykol, Ethylpolyethylenglykol, Propylpolyethylenglykol, Methylpolypropylenglykol, Ethylpolypropylenglykol, Propylpolypropylenglykol, gegebenenfalls aminiert, mit einem Molekulargewicht von 200 bis 12000 g/mol, bevorzugt 300 bis 5000 g/mol, besonders bevorzugt 400 bis 1500 g/mol;

Monosaccharide, z.B. Ribose, Arabinose, Glucose, Mannose, Galactose, Fructose oder Saccharose.

Die Verknüpfung der hydrophilen Verbindungen mit den polymerisierten Fettsäuren bzw. Fettalkoholen erfolgt bevorzugt via Esterbindung bzw. Etherbindung.

Bevorzugte werden von den oben genannten Kondensationsprodukten solche als Solubilisatoren verwendet, die mindestens 50 Gew.-% einer oder mehrerer dimerisierter Fettsäure- oder Fettalkoholderivate der allgemeinen Formel I 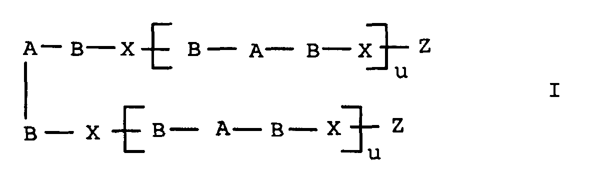 enthalten, in der die Variablen unabhängig voneinander folgende Bedeutung haben:
- A: Rest einer dimerisierten Fettsäure oder eines dimerisierten Fettalkohols mit 22 bis 42 C-Atomen;
- B: -C(=O)- oder -CH₂-;
- X: hydrophiler Baustein aus der Gruppe, bestehend aus Polyalkoholen, Monosacchariden, Alkylenglykolen, Polyalkylenglykolen und aminierten Polyalkylenglykolen;
- Z: OR, NHR, R;
- R: H, C₁-C₂₀-Alkyl, C₁-C₂₀-Alkenyl, C₁-C₁₂-Acyl;
- u: 0 bis 20.

Der Gehalt an erfindungsgemäßen dimerisierten Fettsäure- oder Fettalkoholderivaten in den oben genannten Kondensationsprodukten liegt bei mindestens 50 Gew.-%, bevorzugt zwischen 50 und 100 Gew.-%, besonders bevorzugt zwischen 70 und 100 Gew.-%, ganz besonders bevorzugt zwischen 80 und 100 Gew.-%. Daneben können diese Kondensationsprodukte noch 0 bis 50 Gew.-%, bevorzugt 0 bis 30 Gew.-%, besonders bevorzugt 0 bis 20 Gew.-% monomere sowie trimere und höher kondensierte Fettsäuren enthalten.

Als Alkylreste R seien verzweigte oder unverzweigte C₁-C₂₀-Alkylketten, beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Alkenylreste R seien verzweigte oder unverzweigte C₂-C₂₀-Alkenylketten, beispielsweise Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Acylreste R seien verzweigte oder unverzweigte, gesättigte oder ungesättigte C₁-C₁₂-Acylreste bzw. C₁-C₁₁-Alkylcarbonylreste, beispielsweise Formyl, Methylcarbonyl (Acetyl), Ethylcarbonyl, n-Propylcarbonyl, n-Butylcarbonyl, 1-Methylpropylcarbonyl-, 2-Methylpropylcarbonyl, 1,1-Dimethylethylcarbonyl, n-Pentylcarbonyl, 3-Methylbutylcarbonyl, 2,2-Dimethylpropylcarbonyl, n-Hexylcarbonyl, n-Heptylcarbonyl, n-Octylcarbonyl, 2-Ethylherylcarbonyl, n-Nonylcarbonyl, n-Decylcarbonyl, n-Undecylcarbonyl genannt.

Besonders bevorzugt sind solche dimerisierten Fettsäure- und Fettalkoholderivate der Formel Ia, 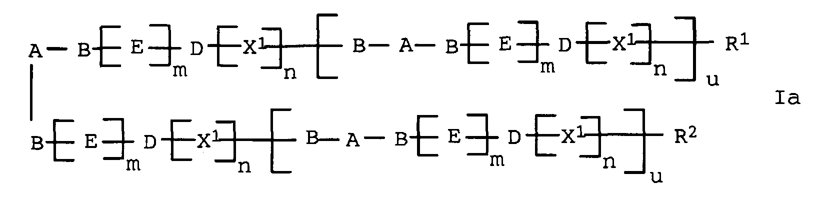 in der die Variablen unabhängig voneinander folgende Bedeutung haben:
- A: Rest einer dimerisierten Fettsäure oder eines dimerisierten Fettalkohols mit 22 bis 42 C-Atomen;
- B: -C(=O)- oder -CH₂-;
- D: -O-;
- E: -N(R³)-Y-;
- X¹: -CH₂-CH₂-O-, -CH₂-CH₂-CH₂-O-, -CH(CH₃)-CH₂-O-, -CH₂-CH₂-CH₂-CH₂-O-, -CH₂-CH(CH₂-CH₃)-O-;
- Y: -CH₂-CH₂-, -CH₂-CH₂-CH₂-, -CH(CH₃)-CH₂-;
- R¹: H, C₁-C₂₀-Alkyl, C₂-C₂₀-Alkenyl, C₁-C₁₂-Acyl;
- R²: H, C₁-C₂₀-Alkyl, C₂-C₂₀-Alkenyl, C₁-C₁₂-Acyl;
- R³: H, C₁-C₂₀-Alkyl, C₂-C₂₀-Alkenyl, [-X¹-]ₙ-R₁;
- m: 0 oder 1;
- n: 1 bis 150;
- u: 0 bis 20.

Als Alkylreste R¹ bis R³ seien verzweigte oder unverzweigte C₁-C₂₀-Alkylketten, beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Alkenylreste R¹ bis R³ seien verzweigte oder unverzweigte C₂-C₂₀-Alkenylketten, beispielsweise Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Acylreste R¹ und R² seien verzweigte oder unverzweigte, gesättigte oder ungesättigte C₁-C₁₂-Acylreste bzw. C₁-C₁₁-Alkylcarbonylreste, beispielsweise Formyl, Methylcarbonyl (Acetyl), Ethylcarbonyl, n-Propylcarbonyl, n-Butylcarbonyl, 1-Methylpropylcarbonyl-, 2-Methylpropylcarbonyl, 1,1-Dimethylethylcarbonyl, n-Pentylcarbonyl, 3-Methylbutylcarbonyl, 2,2-Dimethylpropylcarbonyl, n-Hexylcarbonyl, n-Heptylcarbonyl, n-Octylcarbonyl, 2-Ethylhexylcarbonyl, n-Nonylcarbonyl, n-Decylcarbonyl, n-Undecylcarbonyl genannt.

X¹ sei eine Alkylenglykol-Monomereinheit, ausgewählt aus der Gruppe Ethylenglykol, 1-Methylethylenglykol, n-Propylenglykol, n-Butylenglykol und 1-Ethylethylenglykol.

Die Polyalkylenglykole können dabei aus 1 bis 150 Monomereinheiten, bevorzugt 5 bis 50, besonders bevorzugt aus 7 bis 30 Monomereinheiten zusammengesetzt sein.

Bevorzugte Monomereinheiten für X¹ seien Ethylenglykol und 1-Methylethylenglykol.

Die Polyalkylenglykole sowie die Alkylpolyalkylenglykole können auch mit E = -N(R³)-Y- einseitig aminiert sein, wobei in diesem Fall die Aminogruppe als Säureamid an die polymerisierte Fettsäure gebunden sein kann. Bei der Aminogruppe kann es sich um eine primäre oder sekundäre Aminogruppe handeln, bevorzugt sei dabei der primäre Aminorest.

Ganz besonders bevorzugt sind solche Verbindungen der Formel Ia, in der die Variablen unabhängig voneinander folgende Bedeutung haben:
- A: Rest einer dimerisierten Fettsäure oder eines dimerisierten Fettalkohols mit 30 bis 38 C-Atomen;
- B: -C(=O)- oder -CH₂-;
- D: -O-;
- X¹: -CH₂-CH₂-O-, -CH(CH₃)-CH₂-O-;
- R¹: H, C₁-C₈-Alkyl, C₁-C₈-Acyl;
- R²: H, C₁-C₈-Alkyl, C₁-C₈-Acyl;
- m: 0
- n: 5 bis 50;
- u: 0 bis 15.

Als Alkylreste R¹ und R² seien bevorzugt verzweigte oder unverzweigte C₁-C₈-Alkylketten, besonders bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl genannt.

Als Acylreste R¹ und R² seien bevorzugt verzweigte oder unverzweigte, gesättigte oder ungesättigte C₁-C₈-Acylreste bzw. C₁-C₇-Alkylcarbonylreste, besonders bevorzugt C₁-C₃-Alkylcarbonylreste wie Formyl, Methylcarbonyl (Acetyl), Ethylcarbonyl, n-Propylcarbonyl, n-Butylcarbonyl, 1-Methylpropylcarbonyl-, 2-Methylpropylcarbonyl genannt.

Die bevorzugten hydrophilen Reste Polyethylenglykol und Polypropylenglykol, insbesondere Polyethylenglykol, sowie deren Copolymere können mit ihren endständigen Hydroxylgruppen wiederum an polymerisierten, bevorzugt dimerisierten Fettsäurederivaten bzw. Fettalkoholen kondensiert sein, wobei sich alternierende Blöcke mit bis zu 20 Einheiten, bevorzugt bis zu 15 Einheiten, besonders bevorzugt von 1 bis 5 Einheiten bilden können.

Bei der Verwendung einseitig verschlossener Alkylenglykole bzw. Polyalkylenglykole, bei denen jeweils eine der beiden endständigen Hydroxylfunktion acyliert oder alkyliert (R₁ und R₃ = C₁-C₂₀-Alkyl, C₂-C₂₀-Alkenyl oder C₂-C₁₂-Acyl) vorliegt, erhält man Verbindungen der Strukturformel Ib, 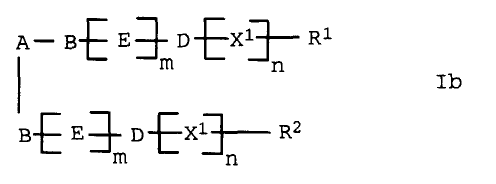 bei denen die Variablen A, B, D, E und X¹ sowie die Indices m und n die bereits oben genannte Bedeutung haben.

Die Herstellung der erfindungsgemäßen polymerisierten Fettsäurederivate erfolgt durch Kondensation der entsprechenden polymerisierten Fettsäuren mit den hydrophilen Verbindungen aus der Gruppe, bestehend aus Polyalkoholen, Monosacchariden, Alkylenglykolen, Polyalkylenglykolen und aminierten Polyalkylenglykolen.

Das Molverhältnis von hydrophilem Baustein zu den polymerisierten Fettsäuren liegt bei 0,5 bis 2,0 mol, bevorzugt 0,7 bis 1,3 mol hydrophiler Baustein pro mol Säuregruppe der Fettsäure.

Die Kondensation kann unter saurer oder basischer Katalyse erfolgen. Als saure Katalysatoren eignen sich Säuren bzw. Lewissäuren, beispielsweise Schwefelsäure, p-Toluolsulfonsäure, phosphorige Säure, hypophosphorige Säure, Phosphorsäure, Methansulfonsäure, Borsäure, Aluminiumchlorid, Bortrifluorid, Tetraethylorthotitanat, Tetrabutylorthotitanat, Zinndioxid, Zinndibutyldilaurat oder deren Gemische.

Als basische Katalysatoren eignen sich Natriummethylat, Natriumethylat, Kaliumcarbonat, Natriumcarbonat, Kaliumtertiärbutylat, Kaliumhydroxid, Natriumhydroxid, Lithiumhydroxid, Magnesiumoxid, Kaliumphosphat.

Der Katalysator wird dabei in Mengen von 0,05 bis 10 Gew.-%, bevorzugt 0,1 bis 5 Gew.-%, bezogen auf die Ausgangsstoffe, eingesetzt.

Die Reaktion kann in Lösungsmittel oder lösungsmittelfrei durchgeführt werden. Die Lösungsmittel sollten bei den Reaktionsbedingungen inert sein.

Als Lösungsmittel eignen sich z.B. Toluol, Xylol, Acetonitril, Hexamethylphosphorsäuretriamid, N,N-Dimethylformamid, N,N-Dimetylacetamid, N-Methylpyrrolidon, Diglyme, Dimethylethylenglykol,Tetrahydrofuran, Dioxan, Ethylencarbonat, Propylencarbonat.

Nach Beendigung der Umsetzung oder während der Umsetzung kann das Lösungsmittel abdestilliert werden.

Die Reaktion wird in der Regel bei Drücken von 5 mbar bis Normaldruck und bei Temperaturen von 60 bis 250°C, bevorzugt 120 bis 200°C durchgeführt. Unter den Reaktionsbedingungen sollte das gebildete Reaktionswasser entfernt werden. Die Reaktionszeiten liegen je nach Bedingungen bei 2 bis 20 Stunden. Reaktionskontrolle erfolgt über IR-Spektroskopie, über die Menge an Reaktionswasser oder über die Bestimmung der Säurezahl.

Die erfindungsgemäßen polymerisierten Fettsäurederivate, insbesondere die polymerisierten Fettsäureester sowie die polymerisierten Fettalkoholderivate der Formel Ia können durch Alkoxylierung der entsprechenden Fettsäuren bzw. Fettalkohole mit Ethylenoxid oder Propylenoxid hergestellt werden. Diese Polyaddition kann unter saurer oder alkalischer Katalyse erfolgen. Bevorzugt sind basische Katalysatoren wie beispielsweise Natriumhydroxid, Kaliumhydroxid, Calciumoxid, Natriummethylat, Kaliumtertiärbutylat, Natrium- oder Kaliumsalze von Carbonsäure.

Der Katalysator wird dabei in Konzentrationen von 0,005 bis 5 Gew.-%, bevorzugt 0,2 bis 1,5 Gew.-%, bezogen auf den polymerisierten Fettalkohol bzw. Säure.

Nach Zugabe des Katalysators können flüchtige Komponenten wie Wasser oder niedere Alkohole destillativ entfernt werden.

Die Alkoxylierug wird bei Temperaturen von 80 bis 160°C, bevorzugt von 100 bis 140°C durchgeführt.

Die so erhaltenen Alkoxylate der polymerisierten Fettsäuren oder Fettalkohole können gegebenenfalls in einem weiteren Schritt mit polymerisierten Fettsäurederivaten erneut verestert werden.

### Anwendungen:

Durch die vorliegende Erfindung werden amphiphile Verbindungen für die Anwendung als Lösungsvermittler für pharmazeutische und kosmetische Zubereitungen sowie für Lebensmittelzubereitungen zur Verfügung gestellt. Sie besitzen die Eigenschaft, schwer lösliche Wirkstoffe auf dem Gebiet der Pharmazie und Kosmetik, schwerlösliche Nahrungsergänzungsmittel, beispielsweise Vitamine und Carotinoide aber auch schwerlösliche Wirkstoffe für den Einsatz in Pflanzenschutzmitteln sowie veterinärmedizinische Wirkstoffe zu solubilisieren.

Überraschend wurde bei den beanspruchten Verbindungen ein gutes Solubilisationsvermögen für pharmazeutische und kosmetische Wirkstoffe gefunden. Ferner werden mit den beanspruchten Verbindungen Anwendungen erhalten, die sich durch eine sehr geringe Hämolyserate, einer nebenwirkungsfreien Verträglichkeit nach parenteraler, oraler und topischer Applikation auf Haut- und Schleimnaut auszeichnen. Die Verbindungen besitzen insbesondere keine Nebenwirkungen durch Wechselwirkungen mit Blutkörperchenmembranen. Nach parenteraler Applikation findet keine bzw. nur eine geringe Histaminfreisetzung statt. Die Solubilisatoren sind aufgrund ihres geringen Molekulargewichts nierengängig.

### Solubilisatoren für Kosmetik

Die Verbindungen der Formel I können als Solubilisatoren in kosmetischen Formulierungen eingesetzt werden. Besonders eignen sie sich als Solubilisatoren für kosmetische Öle. Sie besitzen ein gutes Solubilisiervermögen für Fette und Öle, wie Erdnußöl, Jojobaöl, Kokosnußöl, Mandelöl, Olivenöl, Palmöl, Ricinusöl, Sojaöl oder Weizenkeimöl oder für etherische Öle wie Latschenkieferöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamonöl; Pfefferminzöl, Campferöl etc. oder für Mischungen aus diesen Ölen.

Weiterhin können die erfindungsgemäßen Verbindungen der Formel I als Solubilisatoren für in Wasser schwerlösliche oder unlösliche UV-Absorber wie beispielsweise 2-Hydroxy-4-methoxybenzophenon (Uvinul® M 40, Fa. BASF), 2,2',4,4'-Tetrahydroxybenzophenon (Uvinul® D 50), 2,2'-Dihydroxy-4,4'-dimethoxybenzophenon (Uvinul® D49), 2,4-Dihydroxybenzophenon (Uvinul® 400), 2-Cyano-3,3-diphenylacrylsäure-2'-ethylhexylester (Uvinul® N 539), 2,4,6-Trianilino-p-(carbo-2'-ethylhexyl-1'-oxi)-1,3,5-triazin (Uvinul® T 150), 3-(4-Methoxybenzyliden)-campher (Eusolex® 6300, Fa. Merck), N,N-Dimethyl-4-aminobenzoesäure-2-ethylhexylester (Eusolex® 6007), Salicylsäure-3,3,5-trimethylcyclohexylester, 4-Isopropyldibenzoylmethan (Eusolex ® 8020), p-Methoxyzimtsäure-2-ethylhexylester und p-Methoxyzimtsäure-2-isoamylester sowie Mischungen davon verwendet werden.

Gegenstand der vorliegenden Erfindung sind daher auch kosmetische Zubereitungen, die mindestens eine der Verbindungen der Formeln I oder Ia als Solubilisatoren enthalten. Bevorzugt sind solche Zubereitungen, die neben dem Solubilisator einen oder mehrere schwerlösliche kosmetische Wirkstoffe, beispielsweise die oben genannten Öle oder UV-Absorber enthalten.

Bei diesen Formulierungen handelt es sich um Solubilisate auf Wasser oder Wasser/Alkohol-Basis. Die Verbindung I wird als Solubilisator im Verhältnis von 0,2:1 bis 50:1, bevorzugt 0,5:1 bis 20:1, besonders bevorzugt 1:1 bis 15:1, ganz besonders bevorzugt 2:1 bis 12:1 zum schwerlöslichen kosmetischen Wirkstoff eingesetzt.

Der Gehalt an erfindungsgemäßem Solubilisator in der kosmetischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

Zusätzlich können dieser Formulierung weitere Hilfsstoffe zugesetzt werden, beispielsweise nichtionische, kationische oder anionische Tenside wie Alkylpolyglycoside, Fettalkoholsulfate, Fettalkoholsulfonate, Fettalkoholethersulfate, Fettalkoholethersulfonate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate, Cosolventien wie Ethylenglykol, Propylenglykol, Glycerin u.a..

Als weitere Bestandteile können natürliche oder synthetische Verbindungen, z.B. Lanolinderivate, Cholesterinderivate, Isopropylmyristat, Isopropylpalmitat, Elektrolyte, Farbstoffe, Konservierungsmittel, Säuren (z.B. Milchsäure, Zitronensäure) zugesetzt werden.

Diese Formulierungen finden beispielsweise in Badezusatzpräparaten wie Badeölen, Rasierwässern, Gesichtswässern, Mundwässern, Haarwässern, Eau de Cologne, Eau de Toilette usw. Verwendung.

### Beschreibung der Solubilisierungsmethode:

Bei der Herstellung der Solubilisate für kosmetische Formulierugen können die Verbindungen der Formel I als 100%ige Substanz oder als wäßrige Lösung eingesetzt werden.

Üblicherweise wird der Solubilisator in Wasser gelöst und mit dem jeweils zu verwendenden schwerlöslichen kosmetischen Wirkstoff z.B. den o.g. etherischen Ölen bzw. Parfümölen intensiv vermischt, beispielsweise mittels eines Magnetrührers.

Es kann aber auch der zu verwendende schwerlösliche kosmetische Wirkstoff in einer Schmelze des Solubilisators gelöst werden und anschließend unter ständigem Rühren mit demineralisiertem Wasser versetzt werden.

### Solubilisatoren für pharmazeutische Anwendungen

Die beanspruchten Verbindungen eignen sich für die Verwendung als Solubilisator in pharmazeutischen Zubereitungen jeder Art, die dadurch gekennzeichnet sind, daß sie einen oder mehrere in Wasser schwer lösliche oder wasserunlösliche Arzneistoffe oder Vitamine sowie Carotinoide enthalten. Insbesondere handelt es sich dabei um wäßrige Lösungen bzw. Solubilisate zur oralen oder parenteralen Applikation, wie z.B. Injektionslösungen zur intravenösen, intramuskulären oder subkutaner oder intraperitonealer Applikation.

Desweiteren eignen sich die beanspruchten Verbindungen zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen.

Bei der parenteralen Applikation können neben Solubilisaten auch Emulsionen, beispielsweise Fettemulsionen eingesetzt werden. Auch für diesen Zweck eignen sich die beanspruchten Verbindungen um einen schwerlöslichen Arzneistoff zu verarbeiten.

Pharmazeutische Formulierungen der oben genannten Art können durch Verarbeiten der beanspruchten Verbindungen mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Die Verwendung der erfindungsgemäßen Verbindungen als Lösungsvermittler in pharmazeutischen Zubereitungen erfolgt beispielsweise in der Weise, daß der Wirkstoff in dem Solubilisator, gegebenenfalls unter Erwärmen, dispergiert oder gelöst wird und unter Rühren mit Wasser vermischt wird.

Eine andere Herstellvariante ist das Auflösen des Solubilisators in der wäßrigen Phase, gegebenenfalls unter leichtem Erwärmen und das anschließende Lösen des Wirkstoffs in der wäßrigen Solubilisatorlösung. Das gleichzeitige Auflösen von Solubilisator und Wirkstoff in der wäßrigen Phase ist ebenfalls möglich.

Gegenstand der Erfindung sind daher auch pharmazeutische Zubereitungen, die mindestens eine der Verbindungen der Formeln I oder Ia als Solubilisatoren enthalten. Bevorzugt sind solche Zubereitungen, die neben dem Solubilisator einen in Wasser schwerlöslichen oder wasserunlöslichen pharmazeutischen Wirkstoff, beispielsweise aus den oben genannten Indikationsgebieten enthalten.

Besonders bevorzugt sind von den oben genannten pharmazeutischen Zubereitungen solche, bei denen es sich um parenteral applizierbare Formulierungen handelt.

Der Gehalt an erfindungsgemäßem Solubilisator in der pharmazeutischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

### Solubilisatoren für Lebensmittelzubereitungen

Neben der Anwendung in der Kosmetik und Pharmazie eignen sich die erfindungsgemäßen Verbindungen der Formel I auch als Solubilisatoren im Lebensmittelbereich für schwer wasserlösliche oder wasserunlösliche Nähr-, Hilfs- oder Zusatzstoffe, wie z.B. fettlösliche Vitamine oder Carotinoide. Als Beispiel seien klare, mit Carotinoiden gefärbte Getränke genannt.

### Solubilisatoren für Pflanzenschutzzubereitungen

Die Anwendung der erfindungsgemäßen Verbindungen der Formel I als Solubilisatoren in der Agrochemie kann u.a. Formulierungen umfassen, die Pestizide, Herbizide, Fungizide oder Insektizide enthalten, vor allem auch solche Zubereitungen von Pflanzenschutzmitteln, die als Spritz- oder Gießbrühen zum Einsatz kommen.

In den folgenden Beispielen wird die Herstellung der polymerisierten Fettsäurederivate sowie deren Verwendung als Solubilisatoren näher erläutert.

### Beispiele

### Synthesebeispiele

### Beispiel 1

### Ester aus Dimerfettsäure Pripol® 1009 und Polyethylenglykol (Molgewicht 1000)

36,9 g (0,065 mol) Pripol® 1009 (Fa. Unichema) wurden bei 80°C mit 130,0 g (0,13 mol) Pluriol® E 1000 (Fa. BASF) und 1,7 g Hypophosphorige Säure versetzt und 11h bei 180°C unter N₂-Schutzgas gerührt. Das sich bildende Reaktionswasser wurde abdestilliert.
Ausbeute: 161,0 g
Säure-Zahl (SZ): 4,5 mg KOH/g; OH-Zahl (OHZ): 49 mg KOH/g; Verseifungszahl (VZ): 51 mg KOH/g

### Beispiel 2

### Ester aus Dimerfettsäure Pripol® 1009 und Polyethylenglykol (MG 1500)

28,0 g (0,05 mol) Pripol® 1009 wurden bei 80°C mit 150,0 g (0,1 mol) Pluriol® E 1500 und 1,8 g Hypophosphorige Säure versetzt und 20h bei 180°C unter N₂-Schutzgas gerührt. Das sich bildende Reaktionswasser wurde abdestilliert.
Ausbeute: 167,3 g
SZ: 7,4 mg KOH/g; OHZ: 29 mg KOH/g; VZ: 41 mg KOH/g

### Beispiel 3

### Ester aus Dimerfettsäure Pripol® 1009 und Methylpolyethylenglykol (MG 520)

85,2 g (0,15 mol) Pripol® 1009 wurden bei 80°C mit 156,0 g (0,3 mol) Methylpolyethylenglykol und 2,4 g Hypophosphorige Säure versetzt und 20h bei 180°C unter N₂-Schutzgas gerührt. Das sich bildende Reaktionswasser wurde abdestilliert.
Ausbeute: 235 g
SZ: 6,7 mg KOH/g; OHZ: 13 mg KOH/g; VZ: 78 mg KOH/g

### Beispiel 4

### Ester aus Dimerfettsäure Pripol® 1009 und Methylpolyethylenglykol (MG 750)

28,4 g (0,05 mol) Pripol® 1009 wurden bei 80°C mit 75,0 g (0,1 mol) Methylpolyethylenglykol (MG 750) und 1,0 g Hypophosphorige Säure versetzt und 18h bei 180°C unter N₂-Schutzgas gerührt. Das sich bildende Reaktionswasser wurde abdestilliert.
Ausbeute: 97,1 g
SZ: 7,9 mg KOH/g; OHZ: 2,5 mg KOH/g; VZ: 60 mg KOH/g

### Beispiel 5

### Ester aus Trimerfettsäure Pripol® 1040 und Polyethylenglykol (MG 1000)

38,9 g (mit SZ: 188 mg KOH/g) Pripol® 1040 wurden bei 80°C mit 130,0 g (0,13 mol) Pluriol® E 1000 und 1,7 g Hypophosphorige Säure versetzt und 20h bei 180°C unter N₂-Schutzgas gerührt. Das sich bildende Reaktionswasser wurde abdestilliert.
Ausbeute: 162,2g
SZ: 4,7 mg KOH/g; OHZ: 48 mg KOH/g; VZ: 54 mg KOH/g

### Beispiel 6

### Ester aus Dimerfettsäure Pripol® 1009 und Methylpolyethylenglykol (MG 900)

25,6 g (0,05 mol) Pripol® 1009 wurden bei 80°C mit 81,0 g (0,1 mol) Methylpolyethylenglykol (MG 900) und 1,1 g Hypophosphorige Säure versetzt und 16h bei 180°C unter N₂-Schutzgas gerührt. Das sich bildende Reaktionswasser wurde abdestilliert.
Ausbeute: 101 g
SZ: 8.2 mg KOH/g; OHZ: 3.7 mg KOH/g; VZ: 53 mg KOH/g

### Kosmetische Formulierungen

### Beispiel 7

6 g Solubilisator, hergestellt nach den Beispielen 1 bzw. 4 wurden mit 1 g des in Tabelle 1 aufgeführten etherischen Öls bzw. Parfümöls mittels Magnetrührer innig gemischt. Unter ständigem Rühren wurde mit einer Bürette langsam demineralisiertes Wasser ad 100 g hinzugefügt. Die erhaltenen Formulierungen besaßen folgende Zusammensetzung:
1 Gew.-% etherisches oder kosmetisches Öl,
6 Gew.-% Solubilisator,
93 Gew.-% Wasser

**Tabelle 1**

| Solubilisator | etherisches Öl | Aussehen der Formulierung |
|---|---|---|
| | | |
| Beispiel 1 | Fichtennadelöl | opales Solubilisat |
| Beispiel 1 | Rosmarinöl | opales Solubilisat |
| Beispiel 1 | Lavendelöl | klares Solubilisat |
| Beispiel 1 | After Shave "Minos" der Fa. Drom | opales Solubilisat |
| Beispiel 4 | Latschenkieferöl | klares Solubilisat |
| Beispiel 4 | Lavendelöl | opales Solubilisat |

### Beispiel 8

### Sonnenschutzmittel

25 g Dimerfettsäureester, hergestellt gemäß Beispiel 4 wurden bei ca. 60 °C geschmolzen und 2,5 g des Sonnenschutzmittels Uvinul® T 150 wurden in der Schmelze gelöst. Anschließend wurde eine auf 60°C erwärmte Mischung von 62,5 g Aqua bidest und 10 g Glycerol vorsichtig unter Rühren hinzugetropft. Es bildete sich eine klare Lösung, die nach Erkalten auf Raumtemperatur abgefüllt wurde.

### Pharmazeutische Formulierungen

### Beispiel 9

### Diazepam Injektionslösung

400 mg Dimerfettsäureester gemäß Beispiel 3 wurden in 1578 mg Aqua bidest gelöst. Anschließend wurden 10 mg Diazepam zu der Solubilisatorlösung gegeben und gerührt bis der Arzneistoff gelöst war. Die Lösung wurde mit 2 mg Natriumdisulfit und 10 mg Benzylalkohol konserviert und nach üblichen Methoden sterilfiltriert und in Injektionsfläschen gefüllt.

### Beispiel 10

### 17-β-Estradiol Gelatine Kapseln

100 mg 17-β-Estradiol wurden mit 10 g geschmolzener Dimerfettsäureester gemäß Beispiel 3 und 80 g geschmolzenem PEG 6000 sowie 10 g Ethanol gemischt und anschließend direkt in flüssiger Form in Kapseln gefüllt.

### Beispiel 11

### Orale Ciclosporin Formulierung (flüssig befüllte Kapsel)

100 g Ciclosporin A wurden in 770 g Dimerfettsäureester gemäß Beispiel 4, 100 ml Ethanol und 75 ml Propylengykol gelöst und die viskose klare Lösung wurde anschließend in Kapseln abgefüllt.

### Beispiel 12

### Diazepam Emulsion zur parenteralen Applikation

160 g Dimerfettsäureester gemäß Beispiel 4 wurden in 660 g Aqua bidest gelöst. Das Diazepam (10 g) wurde in einer 1:1 Mischung aus Sojaöl und Miglyol Öl (Ölphase betrug 200 g) dispergiert. Zusätzlich wurden 10 g Sojalecithin eingesetzt, das in der Ölphase gelöst wurde. Die beiden Phasen wurden vordispergiert und anschließend per Hochdrucknomogenisation emulgiert.

### Beispiel 13

### 17-β-Estradiol Tablette

10 g 17-β-Estradiol wurden mit 50 g Dimerfettsäureester gemäß Beispiel 4 geschmolzen. Die Schmelze wurde auf 940 g Ludipress gezogen und das Granulat mit 0,5 g Mg-stearat gemischt. Die erhaltene Mischung wurde anschließend tablettiert.

### Beispiel 14

### Diazepam-haltiges Pulver

10 g Diazepam und 400 g des Dimerfettsäureesters gemäß Beispiel 4 als Solubilisator wurden in Ethanol gelöst. Anschließend wurden 1000 g Sorbitol als Trägerstoff hinzugefügt und ebenfalls gelöst.

Danach wurde das Lösungsmittel entfernt und die Mischung im Vakuum getrocknet.

### Beispiel 15

### Solubilisierende Wirkung am Beispiel 17-β-Estradiol und Clotrimazol

Es wurden 20 Gew.-%ige, wäßrige Solubilisatorlösungen eingesetzt. Die erfindungsgemäßen Solubilisatoren wurden unter leichtem Erwärmen bei Temperaturen bis 65 °C geschmolzen und anschließend mit dem Arzneistoff gemischt. Danach wurde Phosphatpuffer pH 7,0 (USP XXIII) in kleinen Anteilen hinzugefügt und bei Raumtemperatur gerührt bis die Sättigungskonzentration des Arzneistoffs erreicht war. Tabelle 2 zeigt die erzielten Konzentrationen der Arzneistoffe in den einzelnen Solubilisatorlösungen.

**Tabelle 2**

| Verbindung | 17-β-Estradiol | Clotrimazol |
|---|---|---|
| Phosphatpuffer pH 7,0 Vergleich | 0,0*⁾ | 0,0 |
| Sorbitanfettsäureester (Tween® 80) - Vergleich | 0,09 | 0,03 |
| Ethoxyliertes Ricinusöl (Cremophor® EL) - Vergleich | 0,06 | 0,01 |
| Beispiel 1 | 0,14 | 0,25 |
| Beispiel 2 | 0,12 | 0,21 |
| Beispiel 3 | 0,18 | 0,35 |
| Beispiel 4 | 0,18 | 0,33 |
| Beispiel 5 | - | - |
| Beispiel 6 | 0,16 | 0,30 |

| | | |
|---|---|---|
| *⁾ Die Zahlenangaben beziehen sich auf die Menge an solubilisiertem Arzneistoff in Gewichtsprozent. | | |

### Beispiel 16

### Bestimmung der Hämolyseaktivität im RBC-Test

In einem RBC-Test (Red Blood Cell) an Kaninchenerythrozyten wurde die hämolytische Aktivität der beanspruchten Verbindungen getestet (siehe Tabelle 3). Die Inkubationszeit betrug 60 min bei Raumtemperatur.

**Tabelle 3**

| Verbindung | Hämolyse der 1 %-igen Lösungen in Phopsphatpuffer |
|---|---|
| Phosphatpuffer pH 7,0 | keine |
| Sorbitanfettsäureester (Tween® 80) | keine |
| Ethoxyliertes Ricinusöl (Cremophor® EL) | keine |
| Beispiel 1 | keine |
| Beispiel 3 | keine |
| Beispiel 4 | keine |
| Beispiel 6 | keine |

### Beispiel 17

### Verträglichkeit am Hund

Nach intravenöser Injektion einer 5 %-igen wäßrigen Lösung der beanspruchten Verbindungen am Hund wurde die Bluthistaminauschüttung verfolgt (Tabelle 4).

**Tabelle 4**

| Verbindung | 5 min. vor Applikation | 5 min. nach Applikation | 15 min. nach Applikation |
|---|---|---|---|
| Sorbitanfettsäureester (Tween 80) | 3*⁾ | 14142 | 58065 |
| Solutol HS 15 | 5 | 138 | 220 |
| Beispiel 3 | 5 | 10 | 9 |

| | | | |
|---|---|---|---|
| *⁾ Die Zahlenangaben stellen Bluthistaminspiegel in ng/ml dar. | | | |

## Patentansprüche

1. Verwendung von polymerisierten Fettsäure- und Fettalkoholderivaten, hergestellt durch Kondensation von polymerisierten C₁₂-C₁₁₀-Fettsäuren oder C₁₂-C₁₁₀-Fettalkoholen mit hydrophilen Verbindungen aus der Gruppe, bestehend aus Polyalkoholen, Monosacchariden, Alkylenglykolen, Polyalkylenglykolen, aminierten Polyalkylenglykolen, als Solubilisatoren.

2. Verwendung nach Anspruch 1, dadurch gekennzeichnet, daß das Kondensationsprodukt mindestens 50 Gew.-% einer oder mehrerer dimerisierter Fettsäure- oder Fettalkoholderivate der allgemeinen Formel I, 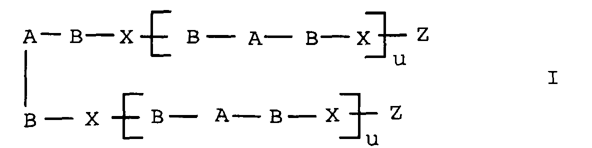 enthält, in der die Variablen unabhängig voneinander folgende Bedeutung haben:
A Rest einer dimerisierten Fettsäure oder eines dimerisierten Fettalkohols mit 22 bis 42 C-Atomen;
B -C(=O)- oder -CH₂-;
X hydrophiler Baustein aus der Gruppe, bestehend aus Polyalkoholen, Monosacchariden, Alkylenglykolen, Polyalkylenglykolen, aminierten Polyalkylenglykolen;
Z OR, NHR, R;
R H, C₁-C₂₀-Alkyl, C₁-C₂₀-Alkenyl, C₁-C₁₂-Acyl;
u 0 bis 20.

3. Verwendung nach Anspruch 1, in der die dimerisierten Fettsäure- und Fettalkoholderivate die folgende allgemeine Formel Ia besitzen, 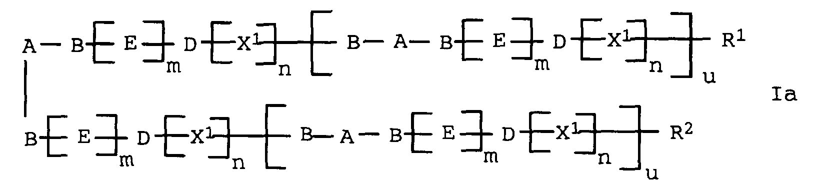 in der die Variablen unabhängig voneinander folgende Bedeutung haben:
A Rest einer dimerisierten Fettsäure oder eines dimerisierten Fettalkohols mit 22 bis 42 C-Atomen;
B -C(=O)- oder -CH₂-;
D -O-;
E -N(R³)-Y-;
X¹ -CH₂-CH₂-O-, -CH₂-CH₂-CH₂-O-, -CH(CH₃)-CH₂-O-, -CH₂-CH₂-CH₂-CH₂-O-, -CH₂-CH(CH₂-CH₃)-O-;
Y -CH₂-CH₂-, -CH₂-CH₂-CH₂-, -CH(CH₃)-CH₂-;
R¹ H, C₁-C₂₀-Alkyl, C₂-C₂₀-Alkenyl, C₁-C₁₂-Acyl;
R² H, C₁-C₂₀-Alkyl, C₂-C₂₀-Alkenyl, C₁-C₁₂-Acyl;
R³ H, C₁-C₂₀-Alkyl, C₂-C₂₀-Alkenyl, [-X¹-]ₙ-R¹;
m 0 oder 1;
n 1 bis 150;
u 0 bis 20.

4. Verwendung von Verbindungen der Formel Ia nach Anspruch 3, in der die Variablen folgende Bedeutung haben:
A Rest einer dimerisierten Fettsäure oder eines dimerisierten Fettalkohols mit 30 bis 38 C-Atomen;
B -C(=O)- oder -CH₂-;
D -O-;
X¹ -CH₂-CH₂-O-, -CH(CH₃)-CH₂-O-;
R¹ H, C₁-C₈-Alkyl, C₁-C₈-Acyl;
R² H, C₁-C₈-Alkyl, C₁-C₈-Acyl;
m 0;
n 5 bis 50;
u 0 bis 15.

5. Verwendung von polymerisierten Fettsäure- und Fettalkoholderivaten nach den Ansprüchen 1 bis 4 als Solubilisatoren in pharmazeutischen und kosmetischen Zubereitungen.

6. Verwendung von polymerisierten Fettsäure- und Fettalkoholderivaten nach den Ansprüchen 1 bis 4 als Solubilisatoren in Lebensmittelzubereitungen.

7. Pharmazeutische Zubereitungen, enthaltend mindestens eine der polymerisierten Fettsäure- und Fettalkoholderivaten gemäß den Ansprüchen 1 bis 4 als Solubilisator.

8. Pharmazeutische Zubereitungen nach Anspruch 7, enthaltend zusätzlich mindestens einen in Wasser schwerlöslichen oder wasserunlöslichen pharmazeutischen Wirkstoff.

9. Pharmazeutische Zubereitungen nach den Ansprüchen 7 und 8, dadurch gekennzeichnet, daß es sich um parenteral applizierbare Darreichungsformen handelt.

10. Kosmetische Zubereitungen, enthaltend mindestens eine der polymerisierten Fettsäure- und Fettalkoholderivaten gemäß den Ansprüchen 1 bis 4 als Solubilisator.

11. Kosmetische Zubereitungen nach Anspruch 10, enthaltend zusätzlich mindestens einen in Wasser schwerlöslichen oder wasserunlöslichen kosmetischen Wirkstoff.

12. Lebensmittelzubereitungen, enthaltend mindestens eine der polymerisierten Fettsäure- und Fettalkoholderivaten gemäß den Ansprüchen 1 bis 4 als Solubilisator.

13. Lebensmittelzubereitung nach Anspruch 12, enthaltend zusätzlch mindestens ein in Wasser schwerlösliches oder wasserunlösliches Vitamin oder Carotinoid.
